# EUROPÄISCHE PATENTSCHRIFT

(11) **EP 0 385 320 B1**
(45) Veröffentlichungstag und Bekanntmachung des Hinweises auf die Patenterteilung: **19.01.1994**
(21) Anmeldenummer: 90103622.8
(22) Anmeldetag: 24.02.1990
(51) Int. Cl.: A61M 1/00, A61M 1/36, A61J 1/05

(54) **Flasche für die Ozonisierung von Blut für die Eigenbluttherapie, von Blutplasma oder Plasmafraktionen**
Bottle for the ozonization of blood for the autohaemotherapy of blood plasma or plasma fractions
Bouteille pour l'ozonisation du sang pour l'autohémothérapie, de plasma sanguin ou de fractions de plasma

(30) Priorität: 27.02.1989 DE 3906034
(43) Veröffentlichungstag der Anmeldung: 05.09.1990
(73) Patentinhaber: Dr. J. Hänsler GmbH, D-76473 Iffezheim (DE)
(72) Erfinder: Viebahn-Hänsler, Renate, Dr., rer. nat., D-7557 Iffezheim (DE); Busch, Karl-Heinz, D-7559 Au am Rhein (DE)
(74) Vertreter: Huss, Carl-Hans, Dipl.-Ing.

(56) Entgegenhaltungen:
- DE-B- 1 071 291
- DE-C- 3 109 691
- FR-A- 989 821
- US-A- 2 406 207
- US-A- 3 647 624

## Beschreibung

Die Erfindung richtet sich auf eine Flasche für die Ozonbehandlung von Blut für die Eigenbluttherapie, von Blutplasma oder Plasmafraktionen. Die erfindungsgemäße Flasche wurde im Hinblick auf die Ozonisierung von Eigenblut entwickelt. Da aber derartige Flaschen in Kliniken allgemein "Plasmaflaschen" genannt werden, wird nachfolgend zur Vereinfachung der Beschreibung nur dieser Begriff verwendet.

Bei der sog. "großen Eigenblutbehandlung mit Ozon", wie sie z.B. in der Einleitung zur Beschreibung der DE-C-31 09 691 beschrieben ist und die eines der Hauptanwendungsgebiete der Ozon-Sauerstofftherapie ist, werden z.Z. 50 bis 100 ml Eigenblut in einer Plasmaflasche mit dem Ozon-Sauerstoffgemisch versetzt und das Reaktionsprodukt in Form einer üblichen Tropfinfusion reinfundiert. Die Zuführung des Gasgemisches in die Flasche geschieht dabei meist über ein im Flaschenstopfen gelagertes, zentrales Röhrchen, das einen Innendurchmesser von etwa 3 bis 4 mm hat. Die Folge ist, daß verhältnismäßig große Blasen des O₂/O₃-Gasgemisches am Röhrchenende austreten. Bei größeren Gasblasen kann aber leicht eine schlechte Verteilung, d.h. eine lokal erhöhte O₃-Konzentration eintreten, die zu einer Teilhämolyse führen kann.

Um die Effektivität zu erhöhen, greift man häufig zu dem Mittel, das O₂/O₃-Gasgemisch unter Druck in das von der Flasche aufgenommene Blut zu geben, wobei O₂ als Druckgas eingesetzt wird. Dadurch wird die Konzentration an gelöstem O₂ wesentlich erhöht, z.B. gegenüber druckloser Einführung um den Faktor 7. Dadurch wird zwangsläufig auch die Gefahr der Radikalbildung erhöht, schon deshalb, weil das Sauerstoffmolekül selbst ein Diradikal ist und deshalb radikalische Folgereaktionen in Gang setzen kann. Vor allem aber werden dabei auch die noch aggressiveren SuperoxidRadikale gebildet, also O₂-Radikal-Anione, die sich durch Aufnahme eines Elektrons im biologischen System bilden können. Dem O₂-Radikal-Anion wird z.B. im entzündlichen rheumatischen Geschehen eine Mediatorfunktion zugeschrieben, die die degenerativen Folgeerscheinungen in den Gelenken einleitet.

Ein weiterer Nachteil des eigentlich zur Effektivitätserhöhung eingesetzten Einführens des O₂/O₃-Gemisches mit Druck ist, daß die Gefahr der Hämolyse durch direkte Druckeinwirkung, also zu große mechanische Belastung der roten Blutkörperchen, weiter erhöht wird.

Weiterhin ist bei der bekannten Art der Einführung des Sauerstoff-Ozon-Gases durch ein zentrales Röhrchen ein optimaler Wirkungsquerschnitt nicht zu erreichen, weil die aus dem freien Ende des Röhrchens austretenden Gasblasen dazu neigen, im Bereich des Röhrchenschafts aufzusteigen, so daß man durch Schütteln der Flasche eine Verteilung der Gasblasen und damit die Beeinflussung des Gesamtblutes zu erreichen versucht, was wiederum die Hämolysegefahr erhöht.

Um diese Nachteile zu beseitigen wurde durch die US-A-2 406 207 eine Flasche für die Ozonisierung von Blut bekannt, bei der durch eine im Flaschenstopfen gelagerte Zuführung das Ozon bis nahe dem Flaschenboden eingeleitet wird, das in Form von Gasblasen am Ende der Zuführung austritt, wobei für die Ozonzufuhr ein sich über dem Boden der Flasche erstreckender Gasverteiler vorgesehen ist. Dieser Gasverteiler, in der Schrift "Filter" genannt, besteht aus einem Maschengitter, dessen Hauptzweck es sein soll, bei dem Einströmen von oben nach unten Fremdpartikel abzufiltern und in das Blut nur sauberes Gaseinströmen zu lassen. Als Nebenzweck ist angegeben, daß das von oben durchströmende Gas danach von der Unterseite des Gitters her zurückströmen muß und dadurch mit dem Blut durchmischt wird. Die Erfahrung hat aber gezeigt, daß ein derartiges Gitter, abgesehen davon, daß es auf der einen Seite als Filter und auf der anderen als Verteiler dienen soll, nicht die gleichmäßige Feinverteilung eines Gases garantieren kann. Dies deshalb, weil das Gas sich immer den Weg des geringsten Widerstandes sucht und demzufolge das gesamte Gas ohne Feinverteilung an der ersten Durchtrittsstelle durchperlen wird.

Die US-A-2 406 207 liegt dem Oberbegriff des Anspruchs 1 Zugrunde.

Aufgabe der vorliegenden Erfindung war nicht nur eine gleichmäßige Verteilung, sondern auch eine Feinverteilung des Gases zu erzielen und zwar so, daß definiert sehr kleine Gasbläschen weitgehend gleicher Größe verteilt über den ganzen Flaschenboden entstehen. Da das Ozon im Bruchteil einer Sekunde eine Reaktion eingeht und nicht physikalisch gelöst wird, kann nur so verhindert werden, daß lokal eine überschießende Reaktion stattfindet, die zur Zerstörung einzelner roter Blutkörperchen führen würde, während in der Nähe oder weiter weg sich im Blut befindende rote Blutkörperchen nicht mehr für eine Reaktion zur Verfügung stehen würden. Die Wegstrecke, die ein O₃-Molekül zurücklegt, ist also extrem kurz, so daß es wichtig ist, daß die Reaktionsoberfläche des Gases möglichst groß ist und sehr gleichmäßig verteilt. Mit einer gleichmäßigen Feinverteilung der wirksamen Komponente O₃ läßt sich auch eine Senkung des Anteils und damit auch eine Senkung des O₂-Anteils zur Verhinderung der Bildung von Radikalen verbinden.

Die Lösung dieser Aufgabe erfolgt mit den in den Ansprüchen definierten Mitteln. Sie ist an einigen, keinen Anspruch auf Vollständigkeit erhebenden Ausführungsbeispielen in den Zeichnungen dargestellt und anhand dieser nachfolgend beschrieben. Es stellen dar:
- Fig. 1: einen Schnitt durch eine Flasche nach der Erfindung in einer ersten Ausführungsform und in etwa natürlichem Maßstab;
- Fig. 2: einen Schnitt durch eine weitere, aus der nach Fig. 1 abgeleiteten Ausführungsform;
- Fig. 3: einen Schnitt gemäß den Fig. 1 und 2 durch eine dritte Ausführungsform;
- Fig. 4: einen Schnitt durch die Flasche nach Fig. 3, geschnitten in der Ebene IV-IV der Fig. 3
1 ist eine Plasmaflasche aus z.B. Glas oder Kunststoff mit verstärktem Hals 2, die durch einen Stopfen 3 verschlossen ist. In den Stopfen 3 ist eine Büchse 4 eingepaßt, durch deren Öffnung 5 das aus dem Ozongenerator kommende O₂/O₃-Gasgemisch in die Flasche eingeführt wird. Im Anschluß an die Öffnung ist in der Büchse 4 ein Gasverteilungshohlraum 7 vorgesehen, der unten durch einen Filter 8 abgeschlossen ist. Die Büchse 4 faßt mit ihrem Ende ein Bündel dünner, starrer Hohlfasern 9 gleicher oder etwa gleicher Länge aus physiologisch inertem Material, z.B. PTFE. Diese Hohlfasern 9 sind so angeordnet, daß ihre Austrittsenden 10 über den ganzen Boden 11 der Flasche verteilt sind. Da die Hohlfasern 9 im Handel für die Herstellung von Injektionsnadeln und Kathetern erhältlich sind, und zwar auch in aufgewickelter Form, können sie von vornherein eine gewisse, spreizende Eigenelastizität aufweisen, so daß sie durch eine ringförmige, axial verschiebbare Fassung 12 so zusammengefaßt werden können, daß die äußersten Fasern noch die untere Ecke der Flasche erreichen, ohne gegen die Flaschenwand anzustoßen.

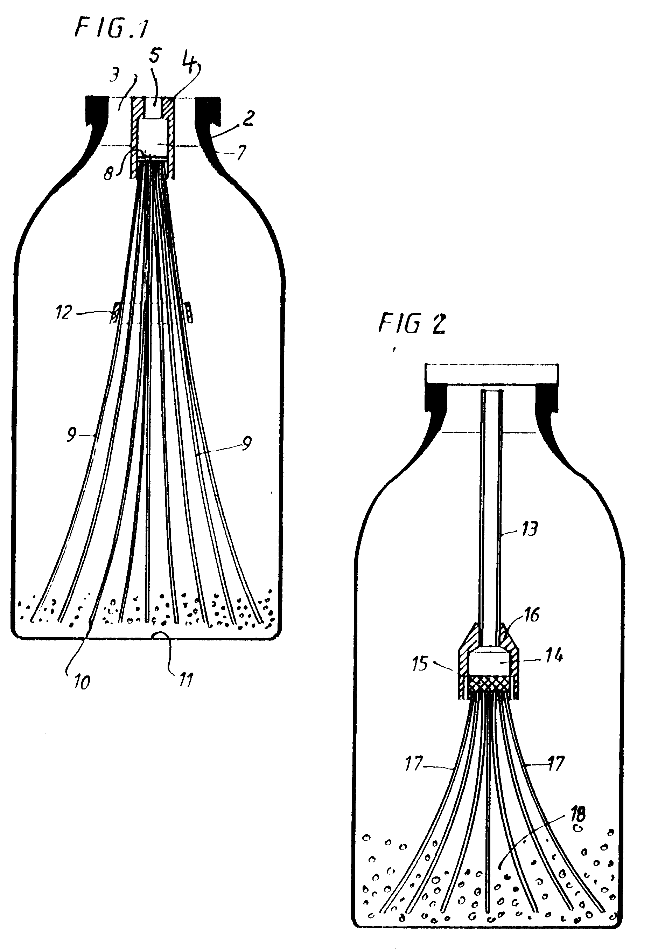

Eine aus Fig. 1 entwickelte weitere Ausführungsform zeigt Fig. 2, die sich von der nach Fig. 1 dadurch unterscheidet, daß das O₂/O₃-Gasgemisch über ein sich nur über einen Teil der Flaschenhöhe erstreckendes, zentrales Rohr 13 dem Verteiler 14 mit Filter 15 zugeführt wird, so daß die im Ende der Büchse 16 gefaßten Hohlfasern 17 nur etwa die untere Flaschenhälfte einnehmen und dann auch keiner Ringfassung 12 gemäß Fig. 1 bedürfen.

Aus den Hohlfasern 9 bzw. 17, deren Innendurchmesser 0,4 bis 0,6 mm beträgt, treten von vornherein sehr kleine Bläschen 18, und zwar Bläschen weitgehend definierter Größe aus, die durch die Anordnung der Faserenden sehr gleichmäßig über den gesamten Flaschenquerschnitt verteilt anfallen und, so ihr Ozon abgebend, nach oben steigen.

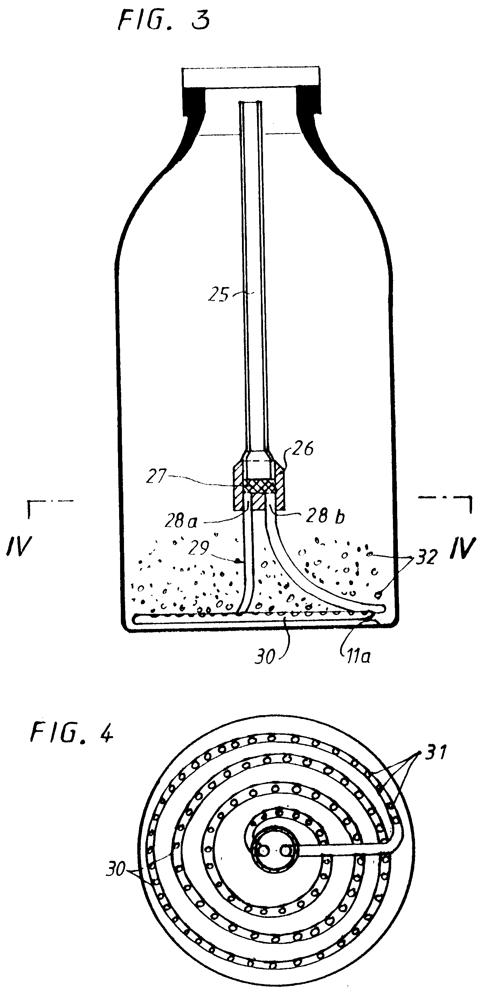

Der obere Teil, der Gasgemischzuführung der Ausführungsform nach den Fig. 3 und 4 ist ähnlich der nach Fig. 2 aufgebaut, d.h. besteht aus einem hinsichtlich Art und Durchmesser vorbekannten Zuführungsrohr 25, das aber unterhalb der waagerechten Flaschenmittelebene in einer Hülse 26 mit Filter 27 endet. Die Hülse 26 ist flaschenbodenseitig geschlossen und nimmt in zwei Bohrungen in ihrem Boden die beiden Enden 28a, 28b eines Verteilerröhrchens 29 von etwa 2 mm Innendurchmesser auf. Dieses ist dicht über dem Boden 11a der Flasche als eine waagerecht liegende Spirale ausgebildet, die mit einer Vielzahl kleiner Öffnungen 31 versehen ist. Auch auf diese Weise werden sich über den gesamten Flaschenboden erstreckende und von diesem aufsteigende sehr kleine O₂/O₃-Bläschen 32 erzeugt. Dadurch, daß die Spirale zweiseitig über die Eintritte 28a und 28b mit dem O₂/O₃-Gemisch beaufschlagt wird, wird die Versorgung der Gesamtspirale gewährleistet.

Was die Ozonkonzentration anbetrifft, so unterscheidet man zwischen der Ozonkonzentration im vom Ozongenerator erzeugten Ozon-Sauerstoff-Gasgemisch in µg/ml und der je Behandlung verabreichten Ozonmenge in µg auf 50 oder 100 ml Blut. Bisher wurde die Empfehlung ausgesprochen, die Ozonkonzentration im Gas nicht über 40 µg/ml zu erhöhen, um wegen der bei Verwendung bekannter Plasmaflaschen auftretenden dicken Gasblasen, eine lokale Hämolyse und lokal überschießende Reaktionen zu vermeiden. Bei Anwendung der erfindungsgemäßen Plasmaflasche läßt sich diese Konzentration wegen der feinen Gasverteilung erheblich senken, da der Wirkungsquerschnitt erhöht ist.

Gleichzeitig kann man auch die Gesamtmenge erniedrigen, da mit Hilfe der Feinverteilung eine bessere Reaktionsausbeute erzielt wird.

## Patentansprüche

1. Flasche für die Ozonisierung von Blut für die Eigenbluttherapie, von Blutplasma oder Plasmafraktionen, in die durch eine in einem Flaschenstopfen (3) gelagerte Zuführung das von einem Generator erzeugte O₂/O₃-Gasgemisch bis nahe dem Flaschenboden eingeleitet wird, das als Gasblasen am Ende der Zuführung austritt, wobei für die Verteilung der Gasblasen ein sich über den Flaschenboden erstreckender Verteiler vorgesehen ist, dadurch **gekennzeichnet**, daß für die Gaszufuhr und gleichzeitige Verteilung des O₂/O₃-Gasgemisches ein Bündel von Hohlfasern (9,17) aus physiologisch inertem Kunststoff eines Innendurchmessers von etwa 0,4 bis 0,6 mm vorgesehen ist, dessen Austrittsendenebene sich über den ganzen Flaschenboden (11) erstreckt.

2. Flasche nach dem Anspruch 1, dadurch **gekennzeichnet**, daß den zusammengefaßten Zuführungsenden der Hohlfasern (9,17) ein Filter (8,15) vorgeschaltet ist, der den unteren Abschluß einer über dem Filter einen Verteilerraum (7,14) aufweisenden Büchse (4,16) bildet.

3. Flasche nach dem Anspruch 1, dadurch **gekennzeichnet**, daß das Bündel aus Hohlfasern (17) nur einen Teil der Höhe der Flasche einnimmt, und daß die seine zusammengefaßten Enden mit Filter aufnehmende Büchse (16) am Ende eines zentralen Zuführungsrohres (13) angebracht ist.

4. Flasche für die Ozonisierung von Blut für die Eigenbluttherapie, von Blutplasma oder Plasmafraktionen, in die durch eine in einem Flaschenstopfen (3) gelagerte Zuführung das von einem Generator erzeugte O₂/O₃-Gasgemisch bis nahe dem Flaschenboden eingeleitet wird, das als Gasblasen am Ende der Zuführung austritt, wobei für die Verteilung der Gasblasen ein sich über den Flaschenboden erstreckender Verteiler vorgesehen ist, dadurch **gekennzeichnet**, daß auf das flaschenbodenseitige Ende eines Zuführungsrohres (25) eine Büchse (26) aufgeschoben oder eingesteckt ist, an deren Boden der Gasblasenverteiler angeschlossen ist, der aus einem mit seinen Enden (28a, 28b) in den Büchsenboden eingesetzten Verteilerröhrchen (29) besteht, das dicht über dem Boden (11a) der Flasche als Spirale (30) mit Öffnungen (31) ausgebildet ist.

## Claims

1. Bottle for the ozonisation of blood for autohaemotherapy of blood plasma or plasma fractions, into which the O₂/)₃ gas mixture created by a generator is introduced through a supply means mounted in a bottle stopper (3) to a point close to the bottom of the bottle, said gas mixture emerging at the end of the supply means as gas bubbles, a distributor extending across the bottom of the bottle being provided in order to distribute the gas bubbles, characterised in that there is provided for the gas supply and simultaneous distribution of the O₂/)3 gas mixture a bundle of hollow fibres (9, 17) made of physiologically inert plastics material, with an internal diameter of approximately 0.4 to 0.6 mm, and whose outlet terminal plane extends across the entire bottom (11) of the bottle.

2. Bottle according to claim 1, characterised in that the combined supplying ends of the hollow fibres (9, 17) are preceded by a filter (8, 15), which forms the lower closure of a bush (4, 16) disposed over the filter and having a distribution space (7, 14).

3. Bottle according to claim 1, characterised in that the bundle of hollow fibres (17) occupies only a portion of the height of the bottle, and in that the bush (16) receiving its combined ends with a filter, is attached at the end of a central supply pipe (13).

4. Bottle for the ozonisation of blood for autohaemotherapy of blood plasma or blood fractions into which the O₂/)3 gas mixture created by a generator is introduced through a supply means mounted in a bottle stopper (3) to a point close to the bottom of the bottle, said gas mixture emerging at the end of the supply means as gas bubbles, a distributor extending across the bottom of the bottle being provided in order to distribute the gas bubbles, characterised in that there is thrust on to or inserted in the end of a supply pipe (25) nearest the base of the bottle a bush (26), to the bottom of which is connected the gas-bubble distributor comprising a distributor tubelet (29) with its ends (28a, 28b) inserted into the base of the bush (26), said tubelet (29) being formed, close above the bottom, (11a) of the bottle as a spiral (30) with openings (31).

## Revendications

1. Bouteille pour l'ozonisation du sang pour l'autohémothérapie, de plasma sanguin ou de fractions sanguines, bouteilles dans laquelle est introduit, par une amenée logée dans un bouchon (3) de la bouteille, un mélange de gaz O₂/O₃ produit par un générateur, qui ressort à l'extrémité de l'amenée sous forme de bulles de gaz, auquel cas pour la répartition des bulles de gaz il est prévu un distributeur s'étendant sur le fond de la bouteille, caractérisée en ce que pour l'amenée de gaz et la répartition simultanée du mélange de gaz O₂/O₃, il est prévu un faisceau de fibres creuses (9, 17) en matière plastique physiologiquement inerte et présentant un diamètre interne d'environ 0,4 à 0,6 mm, faisceau dont le plan des extrémités de sortie s'étend sur tout le fond (11) de la bouteille.

2. Bouteille selon la revendication 1, caractérisée en ce qu'avant les extrémités rassemblées d'admission des fibres creuses (9, 17) il est placé un filtre (8, 15), qui forme la fermeture inférieure d'un fourreau (4, 16) présentant, au-dessus du filtre, une chambre de répartition (7, 14).

3. Bouteille selon la revendication 1, caractérisée en ce que le faisceau de fibres creuses (17) ne prend qu'une partie de la hauteur de la bouteille, et que le fourreau (16), ou sont logés avec le filtre les extrémités regroupées, est mis en place à l'extrémité d'un tube central d'alimentation (13).

4. Bouteille pour l'ozonisation du sang pour l'autohémothérapie, de plasma sanguin ou de fractions sanguines, bouteille dans laquelle est introduit, par une amenée logée dans un bouchon (3) de la bouteille, un mélange de gaz O₂/O₃ produit par un générateur, qui ressort a l'extrémité de l'amenée sous forme de bulles de gaz, auquel cas pour la répartition des bulles de gaz, il est prévu un distributeur s'étendant sur le fond de la bouteille, caractérisée en ce qu'à l'extrémité d'un tube d'alimentation (25), côté fond de la bouteille, est enfilé ou introduit un fourreau (26) au fond duquel est raccordé le distributeur de bulles de gaz qui se compose d'un tube de répartition (29) inséré dans le fond du fourreau par ses deux extrémités (28a, 28b), tube qui est conçu comme une spirale (30) qui se trouve juste au-dessus du fond (11a) de la bouteille et qui présente des orifices (31).
